# EUROPÄISCHE PATENTSCHRIFT

(11) **EP 2 062 543 B1**
(45) Veröffentlichungstag und Bekanntmachung des Hinweises auf die Patenterteilung: **11.07.2012**
(21) Anmeldenummer: 08168933.3
(22) Anmeldetag: 12.11.2008
(51) Int. Cl.: A61B 17/17

(54) **Tibiales Zielgerät für die Doppelkanaltechnik**
Tibial target device for the double channel technique
Appareil cible tibial pour la technique à canal double

(30) Priorität: 23.11.2007 DE 102007057075
(43) Veröffentlichungstag der Anmeldung: 27.05.2009
(73) Patentinhaber: Karl Storz GmbH & Co. KG, 78532 Tuttlingen (DE)
(72) Erfinder: Petersen, Wolf, 48149, Münster (DE); Zantop, Thore, 48161, Münster (DE); Berberich, Sascha, 78532, Tuttlingen (DE); Sauer, Michael, 78532, Tuttlingen (DE); Summerer, Sabine, 78532, Tuttlingen (DE)
(74) Vertreter: Witte, Weller & Partner

(56) Entgegenhaltungen:
- EP-A- 1 449 484
- WO-A-2006/125009
- WO-A-2007/107697
- US-A- 6 019 767
- US-A1- 2002 133 165

## Beschreibung

Die Erfindung betrifft eine Vorrichtung zum Anzielen und Einbringen von Bohrkanälen in die Tibia im Bereich eines Kniegelenkes bei der Rekonstruktion eines vorderen Kreuzbandes, mit einer Handhabe, mit einer einzigen abnehmbar an der Handhabe anbringbaren Führungshülse, deren distales Ende einen ersten Zielpunkt am Knochen darstellt, mit einem vorstehenden Arm, dessen distales Ende einen zweiten Zielpunkt am Tibiaplateau darstellt, wobei der Arm im Bereich dessen distalen Endes eine erste Öffnung aufweist, die mit einer Längsachse der Führungshülse in Ausrichtung steht, so dass ein durch die Führungshülse hindurchgeführter Zieldraht, bei an die Tibia angelegter Vorrichtung, nach Durchdringen des Knochens, auf die erste Öffnung trifft.

Eine derartige Vorrichtung ist aus der US 6 019 767 A1 bekannt.

Ein derartiges tibiales Zielgerät für das vordere Kreuzband ist aus dem Katalog "Arthroskopie, Sportmedizin, Wirbelsäulen-Chirurgie, 2. Ausgabe 1/2005, Seite 71" der Karl Storz GmbH & Co. KG, Tuttlingen, Deutschland, bekannt.

Das vordere Kreuzband des Knies stellt eines der beiden wichtigen Bänder dar, die das Kniegelenk halten. Das zweite Band ist das hintere Kreuzband.

Insbesondere das vordere Kreuzband ist sehr hohen Belastungen ausgesetzt, die dazu führen können, dass das vordere Kreuzband reißt. Das vordere Kreuzband erstreckt sich von dem oberen Plateau (Tibiaplateau) des Unterschenkelknochens (Tibia) und verläuft zur Innenseite des unteren Endes des Oberschenkelknochens (Femur).

Bei der Rekonstruktion des vorderen Kreuzbandes wird dieses entweder durch eine andere natürliche Sehne oder durch ein künstliches Sehnenimplantat ersetzt.

Dazu wird von der Außenseite der Tibia her eine Bohrung eingebracht, die in Höhe des Tibiaplateaus austritt, und zwar an der Stelle, an der das natürliche Kreuzband angewachsen ist. Die Ausrichtung dieser Bohrung ist dabei so, dass diese in etwa der natürlichen Ausrichtung des vorderen Kreuzbandes entspricht, also der Längserstreckung, vom Tibiaplateau ausgehend, zur Innenseite des lateralen Femurkondylus.

Die Bohrung wird dann durch den Femur hindurchgeführt bis diese an dessen Außenseite durchtritt.

In die beiden Bohrkanäle wird anschließend das Sehnenimplantat bzw. das Ersatzband eingesetzt, entsprechend befestigt, so dass es die Funktion des natürlichen vorderen Kreuzbandes einnehmen kann.

Für eine erfolgreiche Rekonstruktion des vorderen Kreuzbandes ist entscheidend, dass der von der Außenseite der Tibia her bewirkte Bohrkanal in einer anatomischen Ausrichtung steht, die der Ausrichtung des Kreuzbandes, bei einer bestimmten Kniestellung, möglichst nahe kommt.

Bei einem arthroskopischen Eingriff steht dem Arzt ein relativ eingeschränktes Gesichtsfeld und außerdem nur relativ wenig Raum im Kniegelenk zur Verfügung, um Manipulationen durchzuführen.

Daher ist der Austrittspunkt des von der Außenseite der Tibia eingebrachten Bohrkanales, der auf dem Tibiaplateau liegt, kaum einzusehen, so dass es sehr schwierig ist, diesen Punkt von der Außenseite her anzuzielen.

Dazu haben sich die eingangs erwähnten tibialen Zielgeräte etabliert. Der distale Endbereich des von der Vorrichtung vorstehenden Armes kann zwischen Tibia und Femur in das Kniegelenk eingebracht werden und dessen Spitze kann an einer Stelle angelegt werden, die in etwa dem Austrittspunkt des tibialen Bohrkanales entspricht. In diesem distalen Endbereich des Armes ist eine Öffnung vorhanden, die in Ausrichtung mit der Längsachse der Führungshülse steht.

Das distale Ende der Führungshülse wird von der Außenseite her an den Knochen angelegt, und zwar in einer solchen Ausrichtung, dass die Längsachse der Führungshülse in etwa der Orientierung der Längserstreckung des vorderen Kreuzbandes entspricht.

Ist das Zielgerät so angesetzt und richtig platziert, wird durch die Führungshülse zunächst ein Zieldraht hindurchgeschoben, der, nachdem er die Tibia durchdrungen hat, auf Höhe des Tibiaplateaus aus dem Knochen austritt und in die Öffnung am distalen Endbereich im Bereich der Spitze einläuft.

Das Zielgerät kann nunmehr abgenommen werden und über den Zieldraht kann ein Hohlbohrer geschoben bzw. geführt werden, der dann den Bohrkanal in der Tibia ausbohrt.

Zur Ausrichtung und Orientierung des Bohrkanals im Femur kann, bei einer bestimmten Winkelstellung des Knies, der bereits in die Tibia eingetriebenen Zieldraht weiter durchgeschoben werden, bis er den Femur durchdringt, so dass dann auch gleich der Bohrkanal im Femur bewerkstelligt werden kann.

Bei genauen anatomischen Betrachtungen des vorderen Kreuzbandes stellt man fest, dass ausgehend vom Tibiaplateau, das vordere Kreuzband in zwei leicht divergierende Bündel aufspaltet, nämlich das sog. anteromediale Bündel (AM) und das posterolaterale Bündel (PL).

Das bedeutet, die beiden Ansatzpunkte dieser Bündel am Femur sind etwas voneinander beabstandet.

Bei der eingangs genannten Operationstechnik wurde diese anatomische Besonderheit nicht berücksichtigt, sondern man hat die beiden Bündel AM und PL als einen gemeinsamen Sehnenstrang angesehen und hat den entsprechenden Bohrkanal so bewerkstelligt, dass er in etwa der Mitte der beiden Bündel liegt.

Da es ein Bestreben in der Rekonstruktionstechnik ist, das Kreuzband möglichst anatomisch exakt zu rekonstruieren, hat sich eine Operationstechnik entwickelt, die dem Umstand Rechnung trägt, dass das vordere Kreuzband, vom Tibiaplateau aus gesehen, entlang der beiden divergierenden Bündel verläuft. Will man hier die Rekonstruktion möglichst nahe den anatomischen Gegebenheiten durchführen, ist es sinnvoll, bei der Rekonstruktion zwei Sehnen oder Transplantate einzusetzen, die sich zum einen entlang der Längsachse des anteromedialen Bündels und zum anderen längs der Längsachse des posterolateralen Bündels erstrecken. Hier ist aber entscheidend, dass die dazu notwendigen zwei Bohrkanäle in einer Ausrichtung, sprich einer Divergenz gesetzt werden können, die möglichst nahe der Längserstreckung des anteromedialen bzw. des posterolateralen Bündels liegen. Diese Technik wird auch als Doppelkanaltechnik bezeichnet.

Aus der WO 2007/107697 A1 ist eine Vorrichtung zum Anzielen und Einbringen von zwei Bohrkanälen in die Tibia im Bereich des Kniegelenkes bei der Rekonstruktion eines vorderen Kreuzbandes bekannt. Es ist eine Handhabe vorgesehen, an der zwei Führungshülsen angebracht sind. Die Mittellängsachsen der Führungshülsen sind so ausgerichtet, dass diese nicht parallel zueinander verlaufen. Eine erste Führungshülse zielt eine erste Öffnung am distalen Ende eines vorstehenden Armes der Vorrichtung an. Die zweite Führungshülse zielt eine zweite Öffnung ebenfalls am distalen Ende des Armes an, die zu der ersten Öffnung beabstandet ist. Dadurch ist es möglich, die sogenannte Doppelkanaltechnik durchzuführen, d.h. durch die beiden Führungshülsen jeweils eine Bohrung zu bewerkstelligen oder einen Zieldraht hindurch zu treiben, die jeweils eine der beiden Öffnungen am distalen Ende erreichen und durch diese hindurch geschoben werden können. Beim Ansetzen der Vorrichtung an eine Tibia können zwei Zieldrähte durch das Tibiaplateau hindurchgeführt werden, wobei die eine Ausrichtung der des anteromedialen Zweiges des vorderen Kreuzbandes und die andere der Ausrichtung des posterolateralen Zweiges des Kreuzbandes entspricht. Somit können mit dieser Vorrichtung mit Hilfe der beiden Hülsen zwei Zieldrähte eingetrieben werden, die dann die Richtung der beiden Zweige des natürlichen vorderen Kreuzbandes haben.

Aus der US 2002/0133165 A1 ist eine Vorrichtung bekannt, mit der zwei parallel zueinander verlaufende Crosspins in einem Knochenkanal bzw. durch diesen hindurch getrieben werden können, in der eine Schlaufe eines Sehnenersatzes aufgenommen ist. Bei dieser Operationstechnik zum Ersatz des vorderen Kreuzbandes wird entweder im Oberschenkel und/oder im Unterschenkel eine sacklochartige Bohrung eingebracht, in die ein Sehnenersatz oder eine an anderer Stelle des Körpers entnommene Sehne eingebracht wird. Aus Stabilitätsgründen wird dabei die Sehne zu einer Schleife gelegt. Um ein axiales Hin- und Herschieben in der Bohrung zu vermeiden, wird im Bereich der Schlaufe quer durch die Bohrung und unter bzw. durch die Schlaufe zumindest ein Crosspin gesetzt, so dass die Schlaufe diesen Crosspin umläuft. Dadurch kann die Sehne auf Zug belastet werden, so dass durch den Crosspin verhindert wird, dass die Sehne wieder aus der Öffnung herausgezogen

wird. Da erhebliche Kräfte einwirken, ist es bekannt geworden, zwei parallel nebeneinander liegende Crosspins einzutreiben.

Dazu ist diese Vorrichtung vorgesehen. Dazu weist sie zwei Hülsen parallel zueinander ausgerichtet auf, durch die die beiden Crosspins hindurchgeschoben werden. In dem in der bereits vorhandenen Bohrung steckenden Teil der Vorrichtung sind Längsnuten ausgespart, durch die die beiden Spitzen der Crosspins durchgetrieben werden können.

Aus der EP 1 449 484 A1 ist eine Führungsvorrichtung für Schrauben oder Bohrer vorgesehen, die mehrere hülsenartige Öffnungen in einem Griffteil aufweist. Die Mittellängsachsen und somit die Ausrichtung dieser hülsenartigen Öffnungen ist jeweils so, dass sie allesamt ein und demselben Zielpunkt an einem distalen Ende der Vorrichtung treffen. Anders ausgedrückt, unabhängig davon, in welche Hülse man einen Bohrer oder ein sonstiges Werkzeug einsteckt oder eintreibt, trifft dieses immer ein und dieselbe Stelle bzw. Öffnung oder Aussparung am distalen Ende der Vorrichtung.

Es ist Aufgabe der vorliegenden Erfindung, ein Zielgerät der eingangs genannten Art dahingehend weiter zu entwickeln, dass mit einem Zielgerät, das nur eine Führungshülse aufweist, zwei Bohrkanäle angezielt und eingebracht werden können, die in ihrer Orientierung möglichst nahe der Längserstreckung des anteromedialen und des posterolateralen Bündels verlaufen.

Erfindungsgemäß wird die Aufgabe dadurch gelöst, dass in einem Abstand zur ersten Öffnung im distalen Endbereich des Armes eine zweite Öffnung vorhanden ist, die nicht in der Längsachse der einzigen Führungshülse liegt, dass ein in der ersten Öffnung aufgenommener Zieldraht, bei von der Vorrichtung abgenommener einziger Führungshülse, in die zweite Öffnung umlegbar ist, und dass nach erneutem Anbringen der einzigen Führungshülse durch diese ein weiterer Zieldraht in die erste Öffnung einbringbar ist.

Durch Vorsehen dieser zweiten Öffnung ist es nun möglich, mit ein und demselben Zielgerät hintereinander zwei Zieldrähte zu setzen, deren Ausrichtung bzw. Orientierung der Orientierung des anteromedialen bzw. des posterolateralen Bündels entspricht.

Dabei wird so vorgegangen, dass zunächst ein erster tibialer Bohrkanal bewerkstelligt wird, der bspw. der Orientierung und Erstreckung des anteromedialen Bündels entspricht. Dazu wird das Zielgerät entsprechend über dessen beide Zielpunkte angesetzt, d.h., das distale Ende der Führungshülse an der Außenseite der Tibia und die Spitze am Tibiaplateau, anschließend wird der erste Zieldraht hindurchgetrieben. Dieser Zieldraht hat dann die Ausrichtung der Längserstreckung des anteromedialen Bündels. Die Führungshülse wird nunmehr vom ersten Zieldraht abgenommen bzw. abgezogen, dabei befindet sich das aus dem Tibiaplateau vorspringende Ende des ersten Zieldrahtes noch in der ersten Öffnung. Der Arm wird nun so bewegt, dass dieses vorstehende Ende aus der ersten Öffnung austritt und dieses überstehende Ende wird nun in die zweite Öffnung eingeschoben. Die Führungshülse wird wieder in die Vorrichtung eingesetzt und erneut wird die Zielvorrichtung an der Tibia angesetzt, und zwar so, dass sich nunmehr die Längsachse der Führungshülse in Richtung des posterolateralen Bündels erstreckt.

Durch die Wahl des Abstandes zwischen der ersten Öffnung und der zweiten Öffnung kann die Lage und die Divergenz vorgegeben werden, die der Lage und der Divergenz der beiden Bündel des vorderen Kreuzbandes entspricht. Nunmehr wird ein zweiter Zieldraht durch die Führungshülse und den Knochen getrieben, bis dieser ebenfalls am Tibiaplateau austritt, allerdings mit einem gewissen Versatz nach medial zu dem bereits gesetzten ersten Zieldraht. Da die Mittellängsachse der Führungshülse mit der ersten Öffnung in Ausrichtung steht, ist bei dem Vorgang sichergestellt, dass sich die beiden Zieldrähte nicht behindern oder aufeinandertreffen. Diese sichere Führung, die Orientierung und Ausrichtung ist auch dadurch gewährleistet, dass beim Setzen des zweiten Zieldrahtes der erste Zieldraht in der zweiten Öffnung aufgenommen ist, somit eine vorbestimmte Orientierung zum zweiten Zieldraht aufweist.

Es ist somit möglich mit ein und demselben Zielgerät durch einen relativ einfachen Vorgang zwei Bohrkanäle bzw. Zieldrähte zu setzen, die sich entsprechend der anatomischen Ausrichtung sowohl des anteromedialen als auch des posterolateralen Bündels erstrecken. Nach Setzen des zweiten Zieldrahtes wird dann die Führungshülse von diesem Draht abgezogen und die Vorrichtung kann aus dem Knie entnommen werden. In der Tibia stecken nun die beiden Zieldrähte und es können anschließend die Bohrvorgänge und auch das Einbringen der tibialen Bohrkanäle durchgeführt werden.

In einer weiteren Ausgestaltung der Erfindung ist die zweite Öffnung nach proximal von der ersten Öffnung beanstandet.

Diese Maßnahme hat den Vorteil, dass beim jeweiligen Anzielen des zweiten Zielpunktes, also der Spitze des Armes, der Blick auf diese Spitze gewährleistet ist, auch dann, wenn in der zweiten Öffnung der zuvor gesetzte erste Zieldraht aufgenommen ist, denn diese vorstehende Spitze ist aus nahezu allen Lagen ersichtlich.

In einer weiteren Ausgestaltung der Erfindung ist die zweite Öffnung als Langlochöffnung ausgebildet.

Diese Maßnahme hat den Vorteil, dass beim Setzen des zweiten Zieldrahts der Abstand zur Mittellängsachse des ersten Zieldrahtes etwas variiert werden kann, je nachdem, ob man den gesetzten ersten Zieldraht am einen oder anderen Ende des Langloches anlegt. Legt man den gesetzten ersten Zieldraht an dem Ende des Langloches an, das in der Nähe der ersten Öffnung liegt, ist der Versatz bzw. der Abstand der Öffnungen geringer als wenn man den gesetzten ersten Zieldraht an dem gegenüberliegenden Ende des Langloches anlegt, das von der ersten Öffnung weiter entfernt wäre.

Diese Endpositionen sind sehr einfach dadurch zu erreichen, dass der Arzt das Zielgerät entweder etwas an sich heranzieht oder von sich wegdrückt, so dass dann der erste gesetzte Zieldraht sich entweder an das eine oder das andere Ende der Langlochöffnung legt. Dies hat auch den Vorteil, dass hier nicht ungewollte oder Fehlstellungen erzeugt werden, die über die anatomischen Variationsmöglichkeiten hinausgehen.

In einer weiteren Ausgestaltung der Erfindung ist die erste Öffnung als Langlochöffnung ausgebildet.

Diese Maßnahme hat den Vorteil, dass die Ausgestaltung als Langlochöffnung das Abnehmen des Armes von dem ersten Zieldraht, nach Setzen des ersten Zieldrahtes, von dessen durch den Knochen durchgetriebenen ersten Endes erleichtert. Dies kann bspw. durch ein seitliches Verkippen der Vorrichtung erfolgen, so dass, wenn das Ende nur wenige Millimeter über das Tibiaplateau übersteht, das schon ausreicht, dass dieses Ende aus der Langlochöffnung austreten kann.

In einer weiteren Ausgestaltung der Erfindung ist die erste Öffnung seitlich offen, so dass ein in der ersten Öffnung aufgenommener erster Zieldraht seitlich aus der Öffnung heraus bewegbar ist.

Diese Ausgestaltung hat den Vorteil, dass durch ein seitliches Verschieben des distalen Endbereiches des Armes der in der ersten Öffnung aufgenommene Zieldraht aus dieser heraustreten kann. Dies ist auch bspw. möglich, wenn der ersten Zieldraht ziemlich weit über das Tibiaplateau hinauseingetrieben worden ist, denn durch die seitliche Öffnung ist das Austreten unabhängig davon möglich, wie weit dieses Ende vorsteht.

In einer weiteren Ausgestaltung der Erfindung beträgt der Abstand der Mittelpunkte von erster und zweiter Öffnung ca. 8-10 mm.

Es hat sich gezeigt, dass dieses Abstandmaß den anatomischen Gegebenheiten eines Menschen Rechnung trägt, um die entsprechende Orientierung von anteromedialem und posterolateralem Bündel zu erzielen.

In einer weiteren Ausgestaltung der Erfindung sind die Öffnungen als durch den Körper des Armes hindurchgehende Bohrungen ausgebildet.

Diese Maßnahmen haben nicht nur den Vorteil, dass diese Öffnungen einfach zu bewerkstelligen sind, sondern erlauben auch das völlige Durchschieben der aus dem Tibiaplateau ausgetretenen Enden der Zieldrähte durch den Körper des Armes hindurch. Es wäre theoretisch auch ausreichend, die Öffnungen nur als Mulden oder Einbuchtungen im Körper auszubilden, allerdings müsste dann sehr darauf geachtet werden, dass die am Tibiaplateau austretenden Enden nur exakt soweit austreten, dass sie in diese Mulden hineinpassen.

Die Handhabung wird also dadurch, dass die Öffnungen als durchgehende Öffnungen ausgebildet sind, noch wesentlich erleichtert. Insbesondere im Zusammenhang mit der zuvor genannten Maßnahme, wonach die erste Öffnung noch seitlich offen ist, ist die Handhabung erheblich erleichtert.

Es versteht sich, dass die vorstehend genannten und die nachstehend zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand eines ausgewählten Ausführungsbeispiels in Zusammenhang mit den beiliegenden Zeichnungen näher beschrieben und erläutert.

Es zeigen:
- Fig. 1: eine perspektivische Ansicht einer erfindungsgemäßen Vorrichtung,
- Fig. 1a: eine vergrößerten Darstellung des in Fig. 1 mit einem Kreis umgrenzten Bereiches,
- Fig. 2: stark schematisiert ein menschliches Bein im Bereich des Kniegelenkes zur Erläuterung der Orientierung des anteromedialen und des posterolateralen Bündels des vorderen Kreuzbandes,
- Fig. 3: den Einsatz der Vorrichtung von Fig. 1 beim Setzen eines ersten tibialen Zieldrahtes, wobei der Übersicht halber der Femur nicht dargestellt ist,
- Fig. 4: eine der Fig. 3 entsprechende Darstellung nach Setzen des ersten Zieldrahtes,
- Fig. 5: eine Situation, nachdem die Führungshülse vom ersten, bereits gesetzten, Zieldrahtes abgenommen ist und dessen über das Tibiaplateau vorstehendes Ende aus der ersten Öffnung herausbewegt und in die zweite Öffnung eingefädelt wurde,
- Fig. 6: eine Situation, bei der ein zweiter Zieldraht gesetzt wurde, und
- Fig. 7: die Tibia mit den beiden gesetzten Zieldrähten nach Entfernen der Vorrichtung.

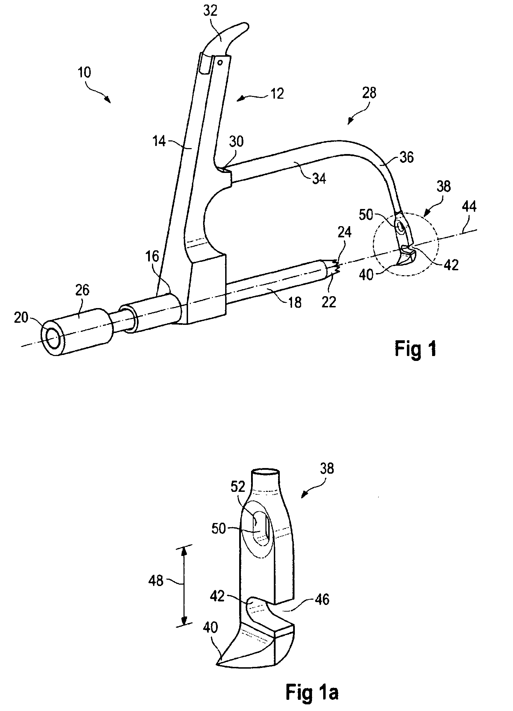

Eine in den Figuren 1 bzw. 1a dargestellte Vorrichtung ist in ihrer Gesamtheit mit der Bezugsziffer 10 bezeichnet.

Die Vorrichtung 10 weist eine Handhabe 12 auf, die einen geradlinigen, lang erstreckten stabförmigen Griff 14 aufweist. An einem Endbereich ist in einer Öffnung 16 eine Führungshülse 18 eingesetzt. Die Führungshülse 18 weist ein Rohr 20 auf, dessen distales Ende 22 mit einem Zackenkranz 24 versehen ist. Am gegenüberliegenden Ende ist die Führungshülse 18 mit einer Klemmhülse 26 versehen. Der Klemmmechanismus ist so ausgestaltet, dass das Drehen der Klemmhülse 26 in einer Richtung eine ortsfeste Halterung der Führungshülse 18 bewerkstelligt, bspw. in der in Fig. 1 darstellten Ausrichtung bzw. Verschiebelage.

Wird die Klemmhülse 26 in die entgegengesetzte Richtung gedreht, kann die Führungshülse 18 längs ihrer Längsachse 44 hin- und herbewegt werden, oder auch vollkommen von dem Griff 14 abgezogen werden, in der Darstellung von Fig. 1 nach links.

Vom stabförmigen Griff 14 springt, etwa auf halber Höhe gesehen, ein Arm 28 vor. Der Arm 28 ist in einer Halterung 30 aufgenommen, und kann durch einen Klemmhebel 32 festgestellt oder entsprechend gelöst, d.h. abgezogen werden.

Der Arm 28 weist einen geradlinigen Abschnitt 34 auf, der sich in etwa parallel zur Führungshülse 18 erstreckt. Der geradlinige Abschnitt 34 geht über eine Krümmung 36 in einen distalen Endbereich 38 über. Am äußeren distalen Ende ist eine Spitze 40 ausgebildet, die dem Zackenkranz 24 der Führungshülse 18 zugewandt ist.

Wie insbesondere aus der vergrößerten Darstellung von Fig. 1a ersichtlich ist, ist im distalen Endbereich 38 in dem Körper des Armes 28 eine erste Öffnung 42 ausgenommen. Die erste Öffnung 42 ist als durch den Körper des Arms 28 vollständig hindurchgehende Öffnung ausgebildet. Seitlich ist die erste Öffnung 42 über eine Nut 46 offen.

Die Mittellängsachse der ersten Öffnung 42 fluchtet mit der Längsachse 44 der Führungshülse 18.

Das heißt, wird von proximal, in der Darstellung von Fig. 1 von der linken Seite her, beispielsweise ein Zieldraht, wie das nachfolgend noch beschrieben wird durch die Führungshülse 18 hindurch geschoben, trifft dieser exakt die erste Öffnung 42.

In einem Abstand 48 zur Mittellängsachse der ersten Öffnung 42 ist eine zweite Öffnung 50 im distalen Endbereich 38 vorgesehen, deren Orientierung gleich wie die der ersten Öffnung 42 ist.

Wie insbesondere aus Fig. 1a zu ersehen, ist die zweite Öffnung 50 als Langloch 52 ausgebildet, wobei sich die längere Achse von distal nach proximal erstreckt.

Ebenfalls ist ersichtlich, dass die zweite Öffnung 50, von der Spitze 40 aus gesehen, proximal von der ersten Öffnung 42 beabstandet ist. Wie insbesondere aus Fig. 1a zu entnehmen ist, ist die zweite Öffnung 50 stark angefast.

Der Abstand 48 beträgt in etwa 8 bis 10 mm.

Die Länge der längeren Achse des Langloches 52 beträgt etwa 8 bis 10 mm.

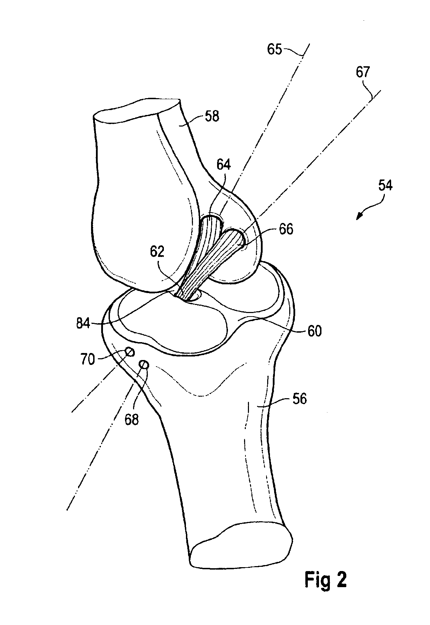

Der Sinn und Zweck dieser Ausgestaltung soll nachfolgend anhand der Figurenfolge von Fig. 2 bis Fig. 7 näher beschrieben und erläutert werden.

In Fig. 2 ist schematisch ein Ausschnitt eines menschlichen Beines 54 im Bereich des Kniegelenkes dargestellt, wobei die Tibia 56, also der Unterschenkelknochen sowie der Femur 58, also der Oberschenkelknochen ersichtlich ist. Vom Tibiaplateau 60 der Tibia 56 erstreckt sich das vordere Kreuzband 62 und zwar dorsal bis hin zur Innenseite des lateralen Femurkondylus.

Es ist zu erkennen, dass das vordere Kreuzband 62, ausgehend vom Tibiaplateau 60 ein anteromediales Bündel 64 und ein posterolaterales Bündel 66 aufweist.

Die entsprechenden Längsachsen 65 und 67 divergieren, ausgehend von dem Tibiaplateau 60 gesehen.

Sinn und Zweck der Vorrichtung 10 ist es nunmehr, in die Tibia 56 Bohrkanäle 68 und 70 einzubringen, deren Orientierung den Längsachsen 65 bzw. 67 des anteromedialen bzw. des posterolateralen Bündels entsprechen.

Wenn man diese beiden Längsachsen 65 und 67 bis auf das Tibiaplateau 60 verfolgt, sieht man, dass diese medial etwas voneinander beabstandet sind und auch etwas divergieren. Demzufolge müssen die Bohrkanäle 68 und 70 so orientiert und bewerkstelligt werden, dass diese dieser anatomischen Ausrichtung entsprechen.

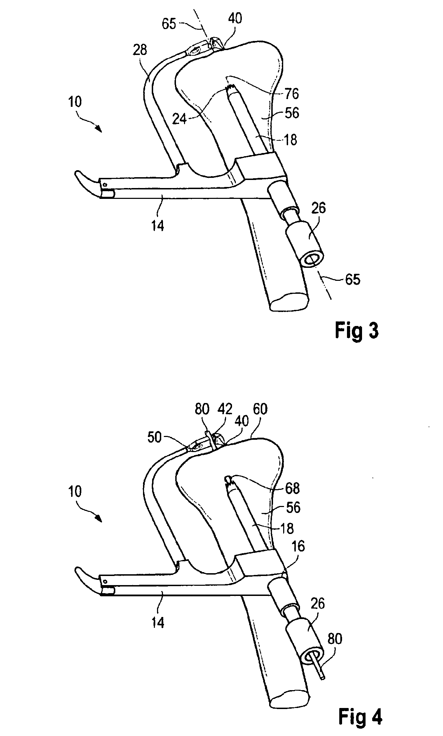

Unter Bezugnahme auf Fig. 3 wird dazu die Vorrichtung 10 in der daraus ersichtlichen Weise an die Tibia 56 angelegt. Der Zackenkranz 54 dient dazu, um fest sitzend einen ersten Zielpunkt 76 an der Außenseite des Knochens zu definieren, der knapp unterhalb der Aufweitung der Tibia liegt. Das distale Ende des Armes 28 wurde durch eine seitliche Öffnung des Knies zwischen Tibia 56 und Femur 58 in die Kniegelenkhöhle eingeführt. Die Spitze 40 stellt einen zweiten Zielpunkt 78 dar. Der Arzt kann diese Orientierung bei einem arthroskopischen Vorgang beispielsweise durch ein Endoskop beobachten. Durch Drehen an der Klemmhülse 26 wird die Vorrichtung 10 in einer festen und sicheren Position gehalten, wobei dies noch durch den Zackenkranz 24 und die etwas in den Knochen eindringende Spitze 40 unterstützt wird.

Wie aus Fig. 4 ersichtlich, wird nunmehr ein erster Zieldraht 80 von proximal nach distal durch die Führungshülse 18 hindurch geschoben, dringt in den Knochen ein, bietet somit den Ausgangspunkt für einen ersten Bohrkanal 68. Der erste Zieldraht 80 wird soweit vorgetrieben, bis er auf Höhe des Tibiaplateaus 60 wieder austritt und dabei in die erste Öffnung 42 eintritt. Die Längsachse des gesetzten ersten Zieldrahtes 80 erstreckt sich dabei längs der Längsachse 65 des anteromedialen Bündels 64, wie das in Fig. 2 angedeutet ist.

Die Klemmhülse 26 wird nun verdreht und die Führungshülse 18 kann sowohl von der Handhabe 12 als auch von dem gesetzten ersten Zieldraht 80 abgezogen werden.

Anschließend wird der distale Endbereich 38 des Armes 28 so bewegt, dass das über das Tibiaplateau 60 vorstehende Ende des ersten Zieldrahtes 80 seitlich aus der ersten Öffnung 42 austritt. Dies vorstehende Ende wird anschließend in die zweite Öffnung 50 eingeschoben. Anschließend wird die Führungshülse 18 wieder in der Handhabe 12 eingeschoben.

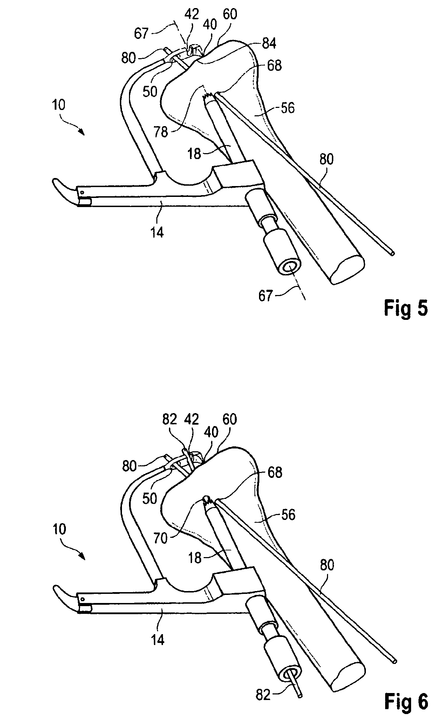

Diese Situation ist in Fig. 5 dargestellt. Hier ist also ersichtlich, dass das über das Tibiaplateau 60 vorstehende Ende des ersten Zieldrahtes 80 in die zweite Öffnung 50 eingeschoben ist, und dass der erste Zieldraht 80 nicht mehr in der Führungshülse 18 aufgenommen ist.

Nunmehr orientiert der Arzt die Vorrichtung 10 derart, dass die Führungshülse 18 bzw. die Öffnung 42 so ausgerichtet wird, dass diese sich in der Längserstreckung der Längsachse 67 des posterolateralen Bündels 66 erstreckt. Dabei wird der Austrittspunkt aus dem Tibiaplateau 60 so gewählt, dass dieser medial versetzt längs des interkondylären Bereichs 84 austritt. Diese Orientierung ist eben noch dadurch erleichtert, dass der erste Zieldraht 80 in der zweiten Öffnung 50 aufgenommen ist. Wie zuvor besprochen ist die zweite Öffnung 50 als Langloch ausgebildet. Zieht der Arzt den stabförmigen Griff 14 etwas nach proximal, also auf sich zu, bewegt sich der erste Zieldraht 80 in der Langlochöffnung 52 so, dass dieser sich an das Ende des Langloches anlegt, das näher zur Öffnung 42 liegt.

Schiebt er hingegen den Griff 14 von sich weg, bewegt sich der erste Zieldraht 80 in Richtung des gegenüberliegenden Endes des Langloches, also an das Ende, das weiter von der ersten Öffnung 42 entfernt ist.

Diese Ausgestaltung erlaubt ihm somit den Abstand etwas zu variieren, um an anatomische Gegebenheiten anpassen zu können.

Befindet sich die Vorrichtung 10 bzw. die Längsachse 44 der Führungshülse 18 in der entsprechenden gewünschten Ausrichtung, also in Ausrichtung der Längsachse 67 des posterolateralen Bündels, wird ein zweiter Zieldraht 82 gesetzt, wie das in Fig. 6 ersichtlich ist.

Der zweite Zieldraht 82 tritt in den Knochen ein und definiert somit die Eintrittsstelle für einen zweiten Bohrkanal 70. Dieser zweite Zieldraht 82 wird definiert in die erste Öffnung 42 geführt, so dass ausgeschlossen ist, dass sich diese beiden Zieldrähte 80, 82 treffen oder behindern.

Anschließend wird die Führungshülse 18 vom zweiten Zieldraht 82 abgezogen, der distale Endbereich 38 des Armes 28 wird dann so bewegt, dass das über das Tibiaplateau 60 überstehende Ende des zweiten Zieldrahtes 82 aus der seitlich offenen ersten Öffnung 42 heraus bewegt wird, anschließend wird dann der distale Endbereich 38 über das entsprechend vorstehende Ende des ersten Zieldrahtes 80 abgezogen und die Vorrichtung 10 kann von der Operationsstelle entnommen werden.

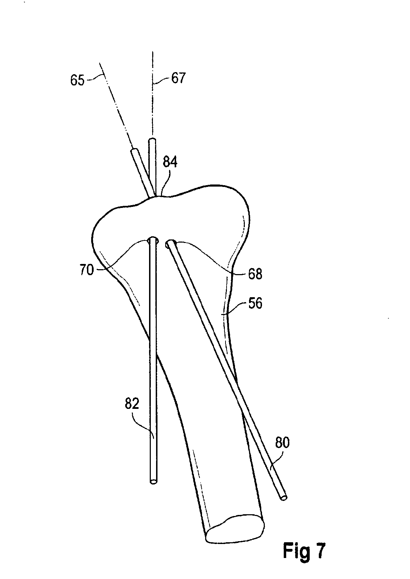

Diese Situation ist in Fig. 7 dargestellt.

Es wurden nunmehr anhand der erfindungsgemäßen Vorrichtung 10 anatomisch richtig orientiert zwei Zieldrähte 80 und 82 gesetzt, deren Längsachsen zum einen in der Längsachse 65 des anteromedialen Bündels und zum anderen in der Längsachse 67 des posterolateralen Bündels erstrecken.

Nunmehr kann über die Zieldrähte 80 und 82 ein Hohlbohrer gebracht werden und die entsprechenden Bohrkanäle können in der Tibia bewerkstelligt werden. Die Zieldrähte 80 und 82 können auch weiter vorgeschoben werden, um beispielsweise in dieser Orientierung durch den Femur bis zu dessen Außenseite hindurchgetrieben zu werden, wobei dies dann in einer Abwinkelstellung des Knies erfolgt, die für die Rekonstruktion günstig ist. Auch hier können dann durch Überschieben entsprechender Hohlbohrer die Kanäle im Femur bewerkstelligt werden.

In die so bewerkstelligten Bohrkanäle 68 und 70 werden dann entsprechend zwei Stränge eines Sehnenersatzes eingeschoben und befestigt, wobei dies durch an sich bekannte Techniken erfolgen kann. Dementsprechend werden dann diese beiden Stränge durch die Bohrkanäle im Femur hindurchgeführt und dort befestigt, wie das ebenfalls an sich bekannt ist. Im Ergebnis erstrecken sich die beiden Sehnenersatzstränge dann längs der Längsachsen des anteriomedialen bzw. des posterolateralen Bündels, so dass eine der Anatomie des Kreuzbandes möglichst nahe kommende Rekonstruktion durchgeführt werden kann.

Die zuvor beschriebene und in der Figurenfolge 3 bis 7 erläuterte Orientierung der Vorrichtung 10 und der Zieldrähte 80 und 82 diente zur Erläuterung der prinzipiellen Anwendung der Vorrichtung an der Tibia 56. Aus darstellerischen Gründen wurde der Femur 58 weggelassen und die anatomisch exakte tibiale Ausgangsstelle des vorderen Kreuzbandes unberücksichtigt gelassen. Entscheidend ist die Knochenbrücke zwischen den tibialen Austrittsstellen der beiden Zieldrähte 80 bzw. 82, d.h. der späteren Bohrkanäle, der Verlauf der beiden Bohrkanal(bzw. Zieldraht)achsen längs des anteromedialen (AM) und des posterolateralen (PM) Bündels, und die Lage der Austrittspunkte aus dem Tibiaplateau entlang des interkondylären Bereiches 84.

## Patentansprüche

1. Vorrichtung zum Anzielen und Einbringen von Bohrkanälen (68, 70) in die Tibia (56) im Bereich eines Kniegelenkes bei der Rekonstruktion eines vorderen Kreuzbandes (62), mit einer Handhabe (12), mit einer einzigen abnehmbar an der Handhabe (12) anbringbaren Führungshülse (18), deren distales Ende (22) einen ersten Zielpunkt (76) am Knochen darstellt, mit einem vorstehenden Arm (28), dessen distales Ende einen zweiten Zielpunkt (78) am Tibiaplateau (60) darstellt, wobei der Arm (28) im Bereich (38) dessen distalen Endes eine erste Öffnung (42) aufweist, die mit einer Längsachse (44) der Führungshülse (18) in Ausrichtung steht, so dass ein durch die Führungshülse (18) hindurch geführter Zieldraht (80, 82), bei an die Tibia (56) angelegter Vorrichtung (10), nach Durchdringen des Knochens, auf die erste Öffnung (42) trifft, **dadurch gekennzeichnet, dass** in einem Abstand (48) zur ersten Öffnung (42) im distalen Endbereich (38) des Armes (28) eine zweite Öffnung (50) vorhanden ist, die nicht in der Längsachse (44) der einzigen Führungshülse (18) liegt, dass ein in der ersten Öffnung (42) aufgenommener Zieldraht (80), bei von der Vorrichtung abgenommener einziger Führungshülse (18), in die zweite Öffnung (50) umlegbar ist, und dass nach erneutem Anbringen der einzigen Führungshülse (18) durch diese ein weiterer Zieldraht in die erste Öffnung (42) einbringbar ist.

2. Vorrichtung nach Anspruch 1, **dadurch gekennzeichnet, dass** die zweite Öffnung (50) nach proximal von der ersten Öffnung (42) beabstandet ist.

3. Vorrichtung nach Anspruch 1 oder 2, **dadurch gekennzeichnet, dass** die zweite Öffnung (50) als Langlochöffnung (52) ausgebildet ist.

4. Vorrichtung nach einem der Ansprüche 1 bis 3, **dadurch gekennzeichnet, dass** die erste Öffnung als Langloch ausgebildet ist.

5. Vorrichtung nach einem der Ansprüche 1 bis 4, **dadurch gekennzeichnet, dass** die erste Öffnung (42) seitlich offen ist, so dass ein in der Öffnung (42) aufgenommener Zieldraht (80, 82) seitlich aus der Öffnung (42) heraus bewegbar ist.

6. Vorrichtung nach einem der Ansprüche 1 bis 5, **dadurch gekennzeichnet, dass** der Abstand (48) der Mittelpunkte von erster (42) und zweiter Öffnung (50) ca. 8 bis 10 mm beträgt.

7. Vorrichtung nach einem der Ansprüche 1 bis 6, **dadurch gekennzeichnet, dass** die Öffnungen (42, 50) als durch den Körper des Armes (28) hindurchgehende Bohrungen ausgebildet sind.

## Claims

1. Device for targeting and introducing bore channels (68, 70) into the tibia (56) in the vicinity of a knee joint during reconstruction of a front cruciate ligament (62), having a handle (12), with a single guide sleeve (18) attachable removably on the handle (12), the distal end (22) of which constitutes a first aiming point (76) on the bone, with a protruding arm (28), whereof the distal end constitutes a second aiming point (78) on the tibial plateau (60), whereby in an area (38) of its distal end the arm (28) has a first opening (42) which is in alignment with a longitudinal axis (44) of the guide sleeve (18) such that a target wire (80, 82) guided through the guide sleeve (18) encounters the first opening (42) after penetrating the bone when the device (10) is placed on the tibia (56), **characterized in that** there is a second opening (50) at a distance (48) from the first opening (42) in the distal end area (38) of the arm (28), which second opening (50) is not within the longitudinal axis (44) of the single guide sleeve (18), further **in that** a target wire (80) received in the first opening (42) can be positioned into the second opening (50) if the single sleeve (18) is removed from the device, and **in that** after mounting the single sleeve (18) again a further target wire can be brought into the first opening (42).

2. Device of Claim 1, **characterized in that** the second opening (50) is at a distance proximally from the first opening (42).

3. Device of Claims 1 or 2, **characterized in that** the second opening (50) is designed as an elongated hole opening (52).

4. Device of any one of Claims 1 through 3, **characterized in that** the first opening is designed as an elongated hole.

5. Device of any one of Claims 1 through 4, **characterized in that** the first opening (42) is open to the side such that a target wire (80, 82) housed in the opening (42) can be moved sideways out of the opening (42).

6. Device of any one of Claims 1 through 5, **characterized in that** the distance (48) of center points of first opening (42) and second opening (50) is about 8 to 10 mm.

7. Device of any one of Claims 1 through 6, **characterized in that** the openings (42, 50) are designed as bores penetrating through the body of the arm (28).

## Revendications

1. Dispositif pour pointer et pratiquer des canaux forés (68, 70) dans le tibia (56) dans la région du genou lors de la reconstruction d'un ligament croisé antérieur (62), avec une manette (12), avec un unique manchon de guidage (18) pouvant être mis en place de manière amovible sur la manette (12) et dont l'extrémité distale (22) représente un premier point de pointage (76) sur l'os, et avec un bras en saillie (28) dont l'extrémité distale représente un deuxième point de pointage (78) sur le plateau tibial (60), sachant que le bras (28) présente dans la région (38) de son extrémité distale une première ouverture (42) qui se trouve en alignement avec un axe longitudinal (44) du manchon de guidage (18), de sorte qu'un fil métallique de pointage (80, 82) acheminé à travers le manchon de guidage (18), lorsque le dispositif (10) est appliqué contre le tibia (56), atteint la première ouverture (42) après avoir transpercé l'os, **caractérisé en ce qu'**une deuxième ouverture (50), présente à une distance (48) de la première ouverture (42) dans la région terminale distale (38) du bras (28), ne se trouve pas dans l'axe longitudinal (44) de l'unique manchon de guidage (18), **en ce qu'**un fil métallique de pointage (80) reçu dans la première ouverture (42) peut être déplacé dans la deuxième ouverture (50) lorsque l'unique manchon de guidage (18) est retiré du dispositif, et **en ce qu'**après avoir à nouveau mis en place l'unique manchon de guidage (18), un autre fil métallique de pointage peut être introduit à travers ce dernier dans la première ouverture (42).

2. Dispositif selon la revendication 1, **caractérisé en ce que** la deuxième ouverture (50) est distante de la première ouverture (42) en direction proximale.

3. Dispositif selon la revendication 1 ou 2, **caractérisé en ce que** la deuxième ouverture (50) est réalisée sous la forme d'une ouverture (52) en trou oblong.

4. Dispositif selon l'une des revendications 1 à 3, **caractérisé en ce que** la première ouverture est réalisée sous forme de trou oblong.

5. Dispositif selon l'une des revendications 1 à 4, **caractérisé en ce que** la première ouverture (42) est ouverte sur un côté, de sorte qu'un fil métallique de pointage (80, 82) reçu dans l'ouverture (42) peut être déplacé latéralement hors de l'ouverture (42).

6. Dispositif selon l'une des revendications 1 à 5, **caractérisé en ce que** la distance (48) entre les centres de la première ouverture (42) et de la deuxième ouverture (50) est d'environ 8 à 10 mm.

7. Dispositif selon l'une des revendications 1 à 6, **caractérisé en ce que** les ouvertures (42, 50) sont réalisées sous la forme de perçages traversant le corps du bras (28).
